(19)

Europäisches
Patentamt
European
Patent Office
Office européen
des brevets

(11) **EP 2 506 004 A2**

(12) **EUROPEAN PATENT APPLICATION**

(43) Date of publication:
**03.10.2012  Bulletin 2012/40**

(21) Application number: **12174429.6**

(22) Date of filing: **14.04.2008**

(51) Int Cl.:
*G01N 29/032* (2006.01)     *G01N 29/22* (2006.01)
*G01N 29/44* (2006.01)     *C12M 1/36* (2006.01)
*G01N 11/16* (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **13.04.2007  GB 0707129**

(62) Document number(s) of the earlier application(s) in accordance with Art. 76 EPC:
**08736970.8 / 2 156 175**

(71) Applicant: **Bioinnovel Limited**
**Babraham**
**Cambridge CB22 3AT (GB)**

(72) Inventor: **The designation of the inventor has not yet been filed**

(74) Representative: **Hall, Graeme Keith**
**Marks & Clerk LLP**
**1 New York Street**
**Manchester, M1 4HD (GB)**

Remarks:
This application was filed on 29-06-2012 as a divisional application to the application mentioned under INID code 62.

(54) **Ultrasonic monitor for a bioreactor**

(57)     A method and apparatus for monitoring the state of a microbiological or animal cell culture, or other bioprocess, wherein the attenuation and in some embodiments the speed, of an ultrasonic wave passed through the culture is used to determine viscous and viscoelastic properties of the culture medium.

Fig. 1

EP 2 506 004 A2

**Description**

Field of the Invention

The invention relates to devices for monitoring a biological culture, for example in the form of a suspension and/or slurry, in a reactor. It has particular application in monitoring parameters that are indicative of the physiological status of microbial cultures, and especially to such devices that monitor continuously.

One application of the invention described herein relates to the monitoring of fermentation broths, especially as manifest through their changing rheological properties. The three main types of industrial fermentation broths comprise fungal, yeast, and bacterial cells.

More generally, the invention addresses process monitoring challenges in colloidal systems, but for simplicity will be described here with reference to fermentation systems. Most multiphase fermentation systems are suspensions comprising microbial cells, protein media particles, gas bubbles and the suspending aqueous phase. Their stability depends on the particle size, sedimentation rate, and extent of microbial cell-cell interactions, e.g. fungal hyphae clumping, cell wall interactions, polymer adsorption, and microbial capsule adherence (bio-film formation). The sedimentation rate is described by the well-known Stokes equation.

Background

On-line measurement of biological processes is desirable in order to make is possible to adjust the process based upon the measurements.

Microorganisms and bio-molecules produced by a biological process are usually present in the form of dispersion in a fluid, normally a liquid.

Oscillatory waves, such as ultrasonic waves, can be used to assess the extent of structure formation by applying small pulses or sinusoidal waves, and measure the attenuation of the wave.

International patent application WO 02/071050 describes the use of acoustic spectroscopy for monitoring food and fermentation systems; in this application, samples of the dispersion to be tested are removed from the reactor Bessel via a bypass loop. Removing samples from the rector vessel means that the acoustic spectroscopy only provides an indication of properties of the culture within the reactor vessel from where the sample is removed, which is usually near a side wall of the reactor. This is usually not a fair representative of the culture as a whole. There is no disclosure in this document of how the state of the culture within the vessel is determined from the acoustic spectroscopy.

Other relevant art known to the applicant may be found in the following patents and patent applications.:

US 6,378357 B1 (2002)
US 2005/0132784 A1
US 2005/0126268 A1
US 2005/0150275 A1

There is no full theoretical treatment that allows physical parameters, such as viscosity, particle size and the volume fraction (as bacteria multiply), to be calculated from acoustic spectroscopy of a typical biological culture. Existing theories break down at high concentrations of suspended particles, because they do not take account of multi-particle scattering of sound waves. They also do not take account of electrokinetic effects.

The bulk (shear) viscosity of microbial suspensions$\eta$, depends on the volume fraction of the particles (the skilled address will be familiar with the Bachelor, Einstein equations on hydrodynamics), and the structure formation due to the effective crosslinking among the cells upon cell division at exponential growth. Moreover, the compressibility of cell clumps expressed as Young modulus, E, and the elasticity of the system in terms of the storage modulus, G' depends on the cell-cell interactions (clumping, effective cross-linking, cell-wall interactions).

Epstein, P. S. and Carhart, R. R., 1953, The Absorption of Sound in Suspensions and Emulsions: I:Water Fog in Air, J. Acoust. Soc. Amer, 25, 553. derive equations for sound waves impinging on a solitary liquid particle; and being absorbed and scattered These authors refer to the "usual methods" - of solving the equations that they derive and these methods can be found in Morse, P. M. and Feshbach, H., 1953, Methods of Theoretical Physics, Volume 2, Chapter 11.3, pp. 1432-1512. Allegra and Hawley.

Allegra, J. R. & Hawley, S. A., 1972, Attenuation of Sound in Suspensions and Emulsions, J. Acoust. Soc. Amer., 51, 1545-1564 apply the method of Epstein and Carhart to the case of an isolated elastic solid particle in a viscous liquid medium. Verdier, C. and Piau, M., 1997, Acoustic Wave Propagation in Two-Phase Viscoelastic Fluids: the Case of Polymer Emulsions, J. Acoust. Soc. Amer., 101, 1868-1876 make a further generalisation of the methods of Epstein and Carhart and Allegra and Hawley, which has become known as "ECAH" theory, to the case where both the liquid medium and the solid suspension have part-liquid-part-solid characteristics known as viscoelasticity. Hipp, A. K., Adjadj, L. P., Storti, G. & Morbidelli, M., 2002, J: Acoust. Soc. Amer, 111, 4, 1549-1551 show that Verdier and Piau re-derived the ECAH results unnecessarily; and that the results of Verdier and Piau actually follow directly from the work of Allegra and Hawley if the viscosity of the liquid and the shear and bulk elastic moduli of the solid phases are replaced by a complex modulus of the type usually used to describe viscoelastic behaviour as described in, for example, Ferry, J. D., 1980, Viscoelastic Properties of Polymers, John Wiley & Sons Inc., ISBN 0471048941.

The ECAH theory can provide reasonable predictions for certain suspensions, however, the theory as currently developed fails to provide accurate predictions for suspensions where particle to particle interactions are significant, such as in a microbial culture. This was reported in Dukhin, A.S. and Goetz, P. J., 2002, Ultrasound for Characterising Colloids, Elsevier, ISBN 0444511644.

Ultrasonic backscattering is known to monitor the growth profile of the microbial culture by determining the particle size distribution. However, this does not provide a particularly information-rich source of data, and systems produced thus far are prone to interference caused by changing and unpredictable flow-regimes within, reactors.

Summary of the Invention

The invention provides in a first aspect a method of monitoring the state of a microbial culture comprising the steps of: passing an ultrasonic wave through the culture; determining attenuation of the ultrasonic wave by the culture; and calculating the effective viscosity of the culture from the attenuation so determined. Changes in the viscosity may be indicative of the state of the microbial culture.

It will be understood that "calculate" as used herein means determine mathematically and/or with computational analysis. Calculation of the viscosity of the culture does not include determining the viscosity by comparing the attenuation of the ultrasonic wave to previous empirical measurements of viscosity.

The state of the microbial culture may be growth rate, the number of damaged cells, the viability and the amount of aggregation (either by clumping in the suspension and/or through the formation of a biofilm)

The method may comprise changing the environmental conditions in the bioreactor in response to the calculation of effective viscosity. For example, if the calculated effective viscosity indicates, excess damage is being caused to the cells, the shear produced in the bioreactor and/or temperature may be altered.

The use of ultrasonic waves to determine the state of a culture is advantageous as the very high frequencies cause little disturbance (small shear) to the sample (i.e. is non-destructive). This is important in a bioreactor wherein damage to the culture should be avoided. Furthermore, as the viscosity is calculated from attenuation changes, the environmental conditions within the bioreactor can be taken into account. Determination of viscosity based on previous empirical measurements is limited in that only a limited number of measurements can be made for specific environmental conditions. Other variables may have to be predetermined, such as specific heat, thermal diffusivity, thermal conductivity, etc of the culture.

Ultrasound attenuation can be used to measure volume viscosity whereas sound velocity and phase angle differences can be used for the evaluation of the contribution of the elastic component of the system.

The culture may comprise a suspension of particles (such as bacteria) in a non-viscoelastic (or at least weakly viscoelastic) liquid medium. The effective viscosity of the culture may be determined using an algorithm, wherein a term is added for a viscoelacticity of the liquid medium. Surprisingly, it has been found that when suspended particles interact, the microbial culture behaves on a macroscopic level as a viscoelastic medium. It has been found that an approximate way to analyse such a system is to assign the viscoelasticity of the culture to the liquid medium (even though this is not the true value of viscoelacticity for the liquid medium). In one embodiment, added to the viscosity of the liquid medium is a term corresponding to the real part of a viscoelastic modulus, such as the storage modulus, $G'$, the loss modulus, $G"$, and $tan(\delta)$. Accordingly, the viscoelastic value determined is indicative of the particle to particle interactions and therefore, the state of the microbial culture.

The algorithm may be an iterative calculation based on the ECAH theory (preferably modified to include an assignment of viscoelasticity to the liquid medium). Alternatively or additionally, the calculation may use finite element analysis, boundary element analysis or other suitable technique.

Attenuation of the ultrasonic wave due to both absorption and scattering due to the particle presence. To simplify the calculation it is desirable that attenuation of the ultrasonic wave due to scattering is negligible. In this way, the effects of scattering of the ultrasonic wave can be ignored for the purpose of the calculation, with attenuation due to absorption dominating. This can be achieved by selecting an appropriate wavelength for the ultrasonic wave.

Attenuation of the ultrasonic wave occurs due to absorption and scattering are separated on the wavelength scale as the wave travels through the sample. The attenuation curve, i.e. attenuation of amplitude is due to the presence of particles, structure, and viscous effects, comprises two characteristic wavelength regions. A "short" wavelength region, wherein the wavelength is less than or of the order of the size of the particles of the culture, and a "long" wavelength region, wherein the wavelength is greater than the size of the particles. In the long wavelength region, attenuation of the ultrasonic wave due to scattering is much smaller than attenuation of the ultrasonic wave due to absorption and can be ignored for the purpose of the calculation.

Accordingly, in one embodiment, the method comprises passing an ultrasonic wave through the culture, wherein the wavelength of the ultrasonic wave is greater than the size of the particles of the culture. In this way, it is not necessary for the calculation to take into account the effects of scattering on the attenuation of the ultrasonic wave. The size of bacteria is typically between $0,5\mu m$ and $100\mu m$ and therefore, the wavelength of the wave should be greater than $100\mu m$ and preferably, $0.5\mu m$.

The frequency of the wave may be is less than 10MHz or more preferably, less than 5MHz.

The invention provides in a second aspect a data carrier having instructions thereon that when executed by a processor causes the processor to receive a measurement of intensity for an ultrasonic wave that has been passed through a microbial culture, determine an attenuation of the ultrasonic wave by the culture; and calculate the viscosity of the culture from the attenuation so determined.

The invention provides in a third aspect apparatus for monitoring the state of a microbial culture in a bioreactor comprising an emitter for generating an ultrasonic wave such that the ultrasonic wave passes through culture in the bioreactor, a detector for measuring the intensity of ultrasonic waves that have passed through the culture, and a computational device arranged to receive signals indicative of the measurement by the detector, determine from the signal the attenuation of the ultrasonic wave; and calculate the viscosity of the culture from the attenuation so determined:

The computational device may determine the effective viscosity of the culture using an algorithm, wherein a term is added for a viscoelasticity of the liquid medium. The computational device may be programmed to calculate the viscosity of the culture from the attenuation based on the ECAH equations, finite element analysis, boundary element analysis or other suitable technique.

The transmitter may be arranged to generate ultrasonic waves having a wavelength less than or of the order of the size of the particles of the culture.

The invention also provides, in a fourth aspect, a method of monitoring the state of a microbial culture in a bioreactor comprising the steps of: locating an emitter for generating an ultrasonic wave in the bioreactor such that the ultrasonic wave passes through culture, locating a detector in the bioreactor for measuring the intensity of the ultrasonic wave that has passed through the culture, signals from the detector used to determine the attenuation of the ultrasonic wave by the culture, the attenuation of the ultrasonic wave being indicative of the state of the microbial culture.

By measuring the state of the culture in situ rather than by removing a sample from the bioreactor, a more accurate determination of the state can be made. Furthermore, it is possible to carry out monitoring of volumes of the culture that are spaced from the walls of the bioreactor. This is advantageous, as the state of the culture will be heterogeneous, with particles tending to aggregate as biofilms on the walls of the reactor. Therefore, sampling near the walls is undesirable as the culture at this position in the reactor will not be representative of the culture as a whole. For example, the culture near the walls may comprise many more viable cells and be more viscous than culture spaced from the walls.

The method may further comprise moving the emitter and/or detector in the bioreactor such that the emitter and/or detector are located at a number of different positions in the bioreactor, and making measurements at those positions. As the culture in a reactor is likely to be heterogeneous, to obtain a true picture of how the process is preceding it is necessary to measure the state of the culture at different locations in the reactor. A map of the culture may be built up from the measurements at diffex-ent locations in the bioreactor.

There is provided, in a fifth aspect of the invention, bioreactor monitoring apparatus for monitoring the state of a microbial culture in a bioreactor comprising: a transmitter for generating an ultrasonic wave and a detector for measuring the intensity of ultrasonic waves and a locating device on which the transmitter and detector are mounted, the device attachable to a bioreactor such that the transmitter and detector are located in the bioreactor.

The locating device may be attachable to the bioreactor such that the positions of the transmitter and/or detector can be adjusted. In this way, the apparatus is able to monitor the culture at more than one position in the bioreactor.

The locating device may comprise a manipulator, such as a robotic arm. The robotic arm may comprise one or more joints that allow positioning of the transmitter and/or detector away from a central axis (in most circumstances, away from a port in the bioreactor through which the robotic arm extends). In this way, monitoring of the culture is not limited to a single axis in the bioreactor.

The robotic arm may comprise one or more drive mechanisms for controlling movement of the arm, the robotic arm arranged such that when the robotic arm is attached to the bioreactor, the drive mechanisms are located external to the bioreactor.

Alternatively, the locating device may be manually operable to adjust the positions of the transmitter and detector.

The locating device is arranged such that it can be inserted into a port of the bioreactor. The industrial standard for bioreactor ports requires a diameter of a few centimetres or less, typically 25 to 30mm. Therefore, the locating device, transmitter and detector should be dimensioned such that they can be inserted into ports of this size.

The apparatus may comprise a probe mounted on the locating device, the probe defining a flow path between an entry orifice and an exit orifice, the transmitter and detector mounted on said probe arranged to transmit an ultrasonic wave through culture in said fluid path. The use of a probe is advantageous as it maintains the alignment of the transducers, to maintain a fixed path distance and maintains the ultrasonic wave within a fixed volume (the flow path) limiting the effects of undesirable reflections of the ultrasonic wave from the walls and anomalies within the bioreactor.

The probe may be of a shape that reduces disruption of the flow in the bioreactor. The probe may comprise an open cylinder shape. In this way, the probe has few edges, protrusions or the like that could cause disruption of the flow.

Preferably, said probe (flow stabiliser) comprises a tube, at least 40mm long with a bore (defining said fluid path) allowing the ultrasonic transmitter and detector to be spaced apart from each other by at least 25mm.

More preferably, at least one of said ultrasonic transmitter and detector is located within the wall of said probe.

The apparatus may comprise a computational device arranged to cause the transmitter to generate ultrasonic waves and receives signals from the detector.

More preferably also, at least one of said ultrasonic transmitter and detector is located at an end region of said probe, and operably coupled thereto. The computational device may determine from the signal the attenuation of the ultrasonic wave and calculate the viscosity of the culture from the attenuation so determined

In apparatus according to the invention it preferably further comprises one or more channels configured to extend, in use, outside a bioreactor containing a microbial culture to be monitored, said channels communicating with said probe, whereby said ultrasonic transmitter and/or detector may be reversibly mounted on the probe though said channel (s) from outside the bioreactor.

The transmitter and detector may comprise transducers. The material for the transducers is chosen such that the transducers can withstand the environmental conditions within the bioreactor. For example, a typical sterilisation temperature is 120°C. Therefore, the transducers need to be able to operate with high resistance, high sensitivity and high time stability at these elevated temperatures, An example of such a material is a piezoelectric transducer such as a lead Zirconate Titanate transducer. The transducer may have a coupling Factor of 80-90, a permittivity of 30-50 F/m, open and short circuit sensitivity of 64dB and 65dB, curie point of 360-370°C and a temperature hysteresis ( which refers to the changes in the coupling factors and relative dielectric constants) with a required range of -9 to -10% for the coupling factor and 5-10% for the dielectric constant.

Included within the scope of the invention is apparatus substantially as described herein, with reference to and as illustrated by any appropriate combination of the accompanying drawings.

Brief Summary of the Drawings

The invention will be described, by example only, with reference to the accompanying drawings, in which:-

**Figure 1** is a schematic illustration of an embodiment of apparatus in accordance with the invention;

**Figure 2** is a perspective view of a bioreactor having therein apparatus in accordance with the embodiment of the invention;

**Figure 3a** is a perspective view of the apparatus shown in the Figure 2 without the bioreactor vessel;

**Figure 3b** is perspective view of an alternative embodiment of the apparatus;

**Figure 4a** is a perspective view of an alternative embodiment of a locating device in accordance with the invention;

**Figure 4b** is a perspective view of an alternative embodiment of a locating device in accordance with the invention;

**Figure 5** illustrates a probe according to an embodiment of the present invention having a cylindrical section;

**Figure 6** illustrates another embodiment of a probe according to the invention having a rectangular-section;

**Figure 7** illustrates a further embodiment of a probe according to the present invention having channels extending outside a bioreactor:

**Figures 8-10** illustrate, in cross-section, embodiments of sensors according to the present invention;

**Figure 11** is a graph illustrating the attenuation of ultrasound by a starch solution compared to water;

**Figure 12** is a graph illustrating ultrasonic pulse echo characterisation of a starch solution, compared to water; and

**Figure 13** is a graph illustrating the change in viscosity of a culture of *Streptomyces venezualae* with time, together with a change in optical density.

Description of Preferred Embodiments

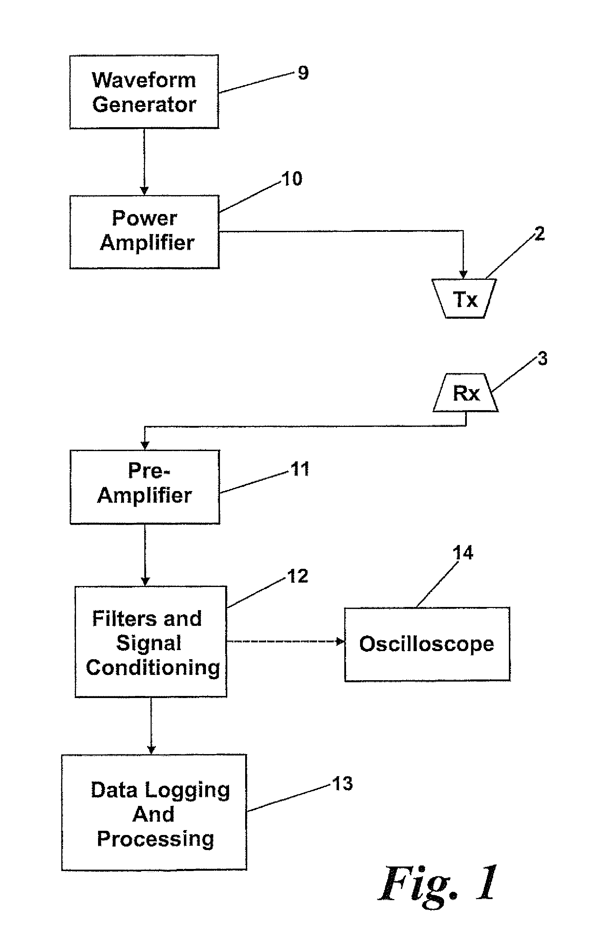

Referring to Figure 1, an embodiment of apparatus for monitoring the state of a microbial culture in a bioreactor is shown. The apparatus comprising an ultrasonic transmitter 2 for generating an ultrasonic wave and a detector 3 for measuring the intensity of ultrasonic waves. The transmitter 2 and detector 3 are arranged in the bioreactor (not shown) such that the ultrasonic wave passes through culture in the bioreactor between the transmitter 2 and detector 3. A waveform generator 9 generates an electrical signal corresponding to the wave, or pulse train required, as will be discussed in more detail below. The electrical signal is amplified by amplifier 10 and transmitter 2 converts the electrical signals into an ultrasonic wave.

The signal from the ultrasonic detector 3 is passed to a pre-amplifier 11 to amplify the received signal. If required, signal-conditioning circuitry 12 may be employed to remove noise from the amplified signal.

This clean signal is then passed to a computational device, for example data logging and processing means 13 such as a computer. The computational device 13 is arranged to determine from the received signal the attenuation of the ultrasonic wave; and calculate the viscosity of the culture from the attenuation so determined, in accordance with the technique described below. If require, the received signal may be displayed on an oscilloscope 14 where it may also be compared with the shape of the input wave 4.

In use, the ultrasonic transmitter 2 may be excited to cause the propagation of an ultrasonic wave or pulse of typically 1-10MHz to be transmitted through the culture, for example a broth fluid, in the bioreactor.

The inventor has found that a frequency of approximately 5MHz (say 4.5-6MHz) is particularly advantageous for the characterisation of fungal broths.

The wave or train of pulses may be propagated continuously for a predetermined time, typically between 1 and 20 seconds. In the case of dilute broths, i.e. Newtonian & shear thinning fluids (typically having a volume fraction of suspending bio-mass particles less than 10%), the wave will be propagated continuously for the determination of the attenuation coefficient as only the viscous contribution needs to be estimated. For more viscous broths, the elastic properties have to be considered, and hence the sound velocity needs to be determined alongside with the attenuation to calculate G', G"& tan($\delta$) so the wave will be applied as a pulse. In the pulse situation (rather than a continuous wave), the attenuation is monitored by the amplitude of the received pulse compared to the emitted pulse. Velocity is measured by the time of flight (TOF) from the pulse being sent to the time it is received using the respective graphs from captured signals. The data may be processed and plotted on a personal computer using e.g. MATLB$^®$ code.

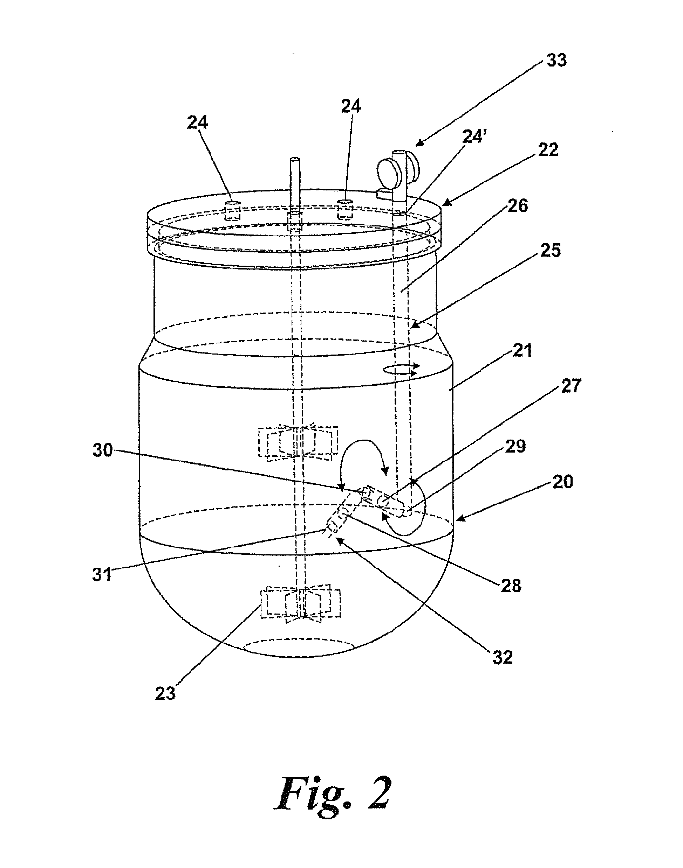

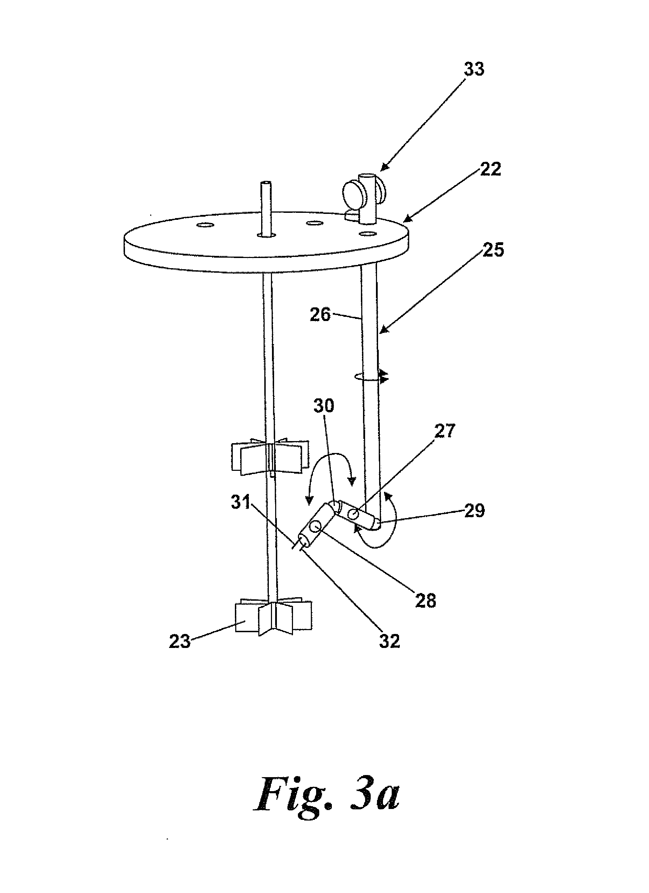

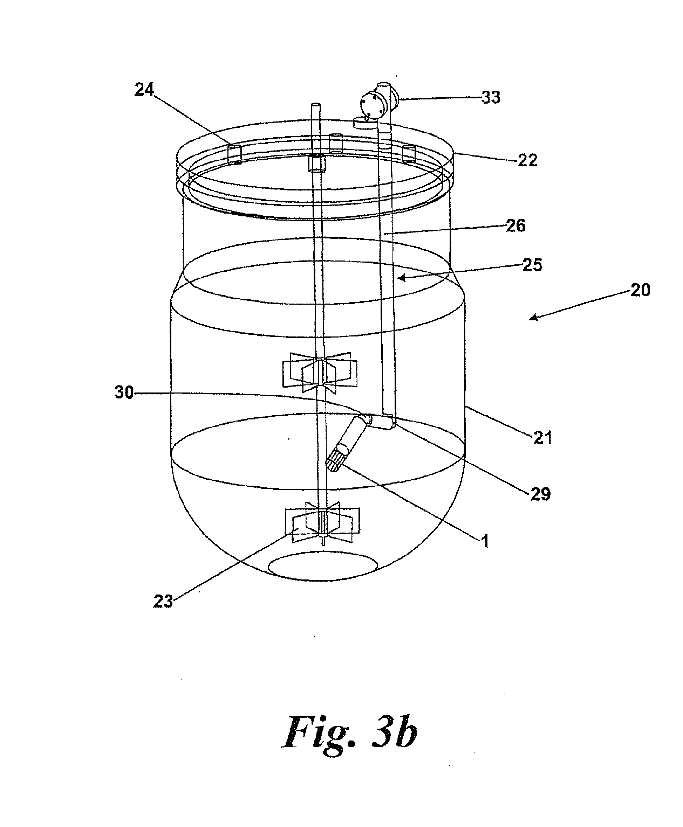

Figure 2 and 3 show apparatus for monitoring a microbial culture in a bioreactor 20. The bioreactor 20 comprises a vessel 21 capped by a head plate 22. Carried by the head plate 22 is an impeller 23 (for example, a Ruston impeller) for stirring the culture in vessel 21. The head plate 22 is provided with a number or ports 24 therein. Inserted in one of the ports 24' is a robotic arm 25.

A locating device, in this embodiment a robotic arm 25, is attached to the bioreactor such that the positions of the transmitter 2 and detector 3 can be adjusted.

The robotic arm 25 comprises a central shaft 26 joined to two further shafts 27 and 28 by elbow joints 29, 30. Located at the end of shaft 28 are transducers 31 and 32 that act as transmitter 2 and detector 3 in the apparatus. The central shaft 26 is able to rotate about its longitudinal axis and to be moved upwardly and downwardly within the reactor to alter the depth of the transducers 31, 32 in the reactor 20. The elbow joints 29, 30 can pivot to position the transducers 31, 32 away from a longitudinal axis of the central shaft 26 such that measurements of the culture at different places within the reactor 20 can be made. A drive mechanism, such as electronic motors 33, located externally of the reactor 20, control operation of the robotic arm 25. In this embodiment, the transducers are made of Zirconate Titanate material.

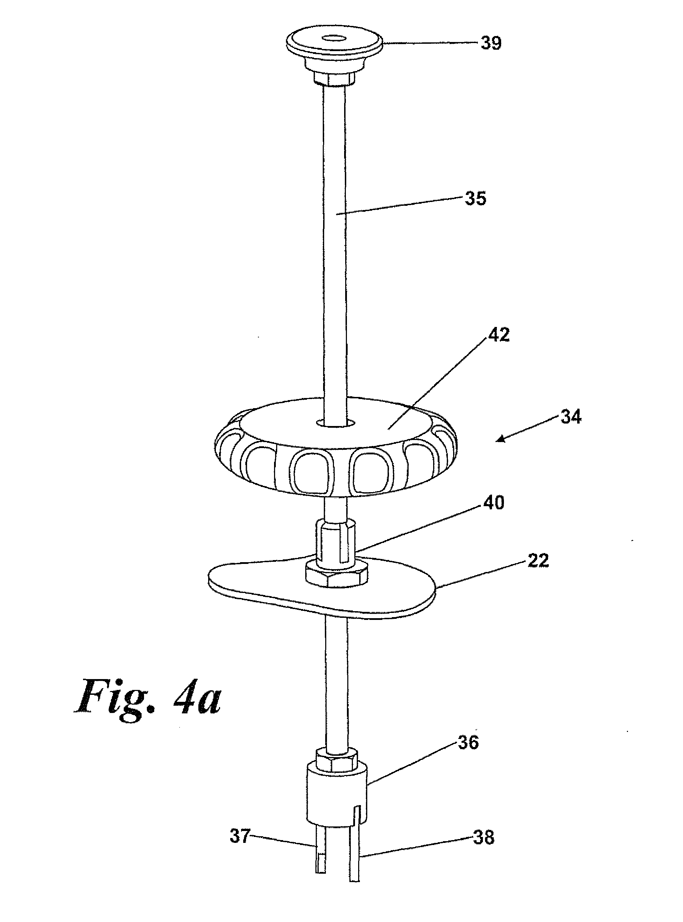

Figure 4a shows an alternative embodiment in which the robotic arm is replaced with a manually operable manipulator 34. This manually operable manipulator comprises a shaft 35 having, at one end, a sensor mount 36 on which the transducers 37, 38 of the transmitter 2 and detector 3 are mounted and at the other end a lifting knob 39 to be gripped by a user when operating the manipulator. The manipulator 34 is inserted through a port 40 in the head plate 22 of the bioreactor 20 and a collet fixing 41 is inserted in the port 40 around the shaft 25. Mounted on the shaft 35 is a wheel 42 that is arranged to co-operate with the collet fixing 41 to form a fluid tight seal and fix the shaft 25 is place. Accordingly, in order to adjust the position of the transducers 37, 38 in the reactor 20, the collet 41 must first be released through operating wheel 42 before the shaft is raised or lowered, typically through using knob 39.

In the above embodiments, the transducers 37 and 38 are shown as extending from the end of the manipulator (robotic arm 25 or manual operated manipulator 34). However, it is preferable that the transducers 37 and 38 are housed within a sensor probe as now described.

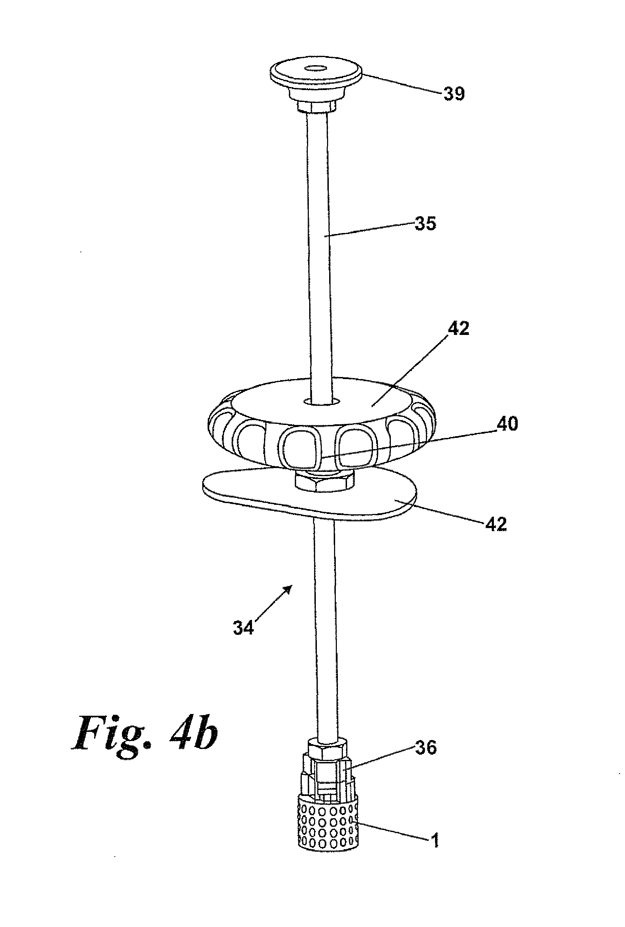

Figures 3b and 4b are examples of such embodiments, wherein a probe 1 housing the transmitter and detector is mounted on the robotic arm 25 or the sensor mount 36. This probe is described in more detail below.

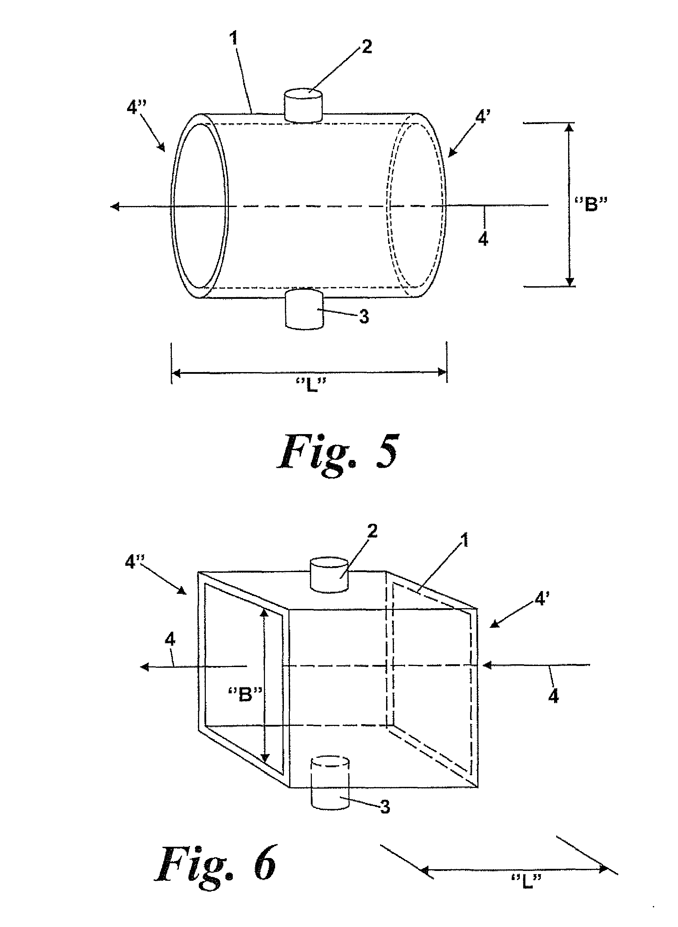

Figure 5 illustrates, in perspective view, a probe (flow stabiliser) 1. The probe 1 defines a flow path 4 (indicated by the arrow 4 running through bore of the probe 1) between an entry orifice 4' and an exit orifice 4". In this embodiment, the probe 1 is in the form a cylindrical tube having a length (identified as "L") of 50mm and a bore (identified by "B") of 30mm, Attached to one side of the tube is an ultrasonic transmitter 2 attached to the outside of the probe 1 on the opposite side is an ultrasonic detector 3.

In use, the probe 1 is mounted on the distal end of the locating device and positioned by the locating device such that the flow path 4 is preferably parallel to the flow of a microbial culture as it is agitated within a bioreactor vessel 20, to create minimum disturbance of the flow, and to avoid the formation of eddies as the sample flows through the probe 1. In use, a signal is fed to the ultrasonic transmitter 2 causing an ultrasonic wave to propagate from the transmitter 2 to the detector 3 across the sample flowing within the probe 1. The probe 1 thus maintains a constant ultrasonic path length and protects the ultrasonic beam from turbulence.

Figure 6 illustrates an alternative configuration of the probe 101 where the probe 101 has the form of a square cross-section tube. Again, the ultrasonic transmitter 2 and ultrasonic detector 3 are mounted on opposite sides of the probe 1, their separation defined by the bore "B" of the tube.

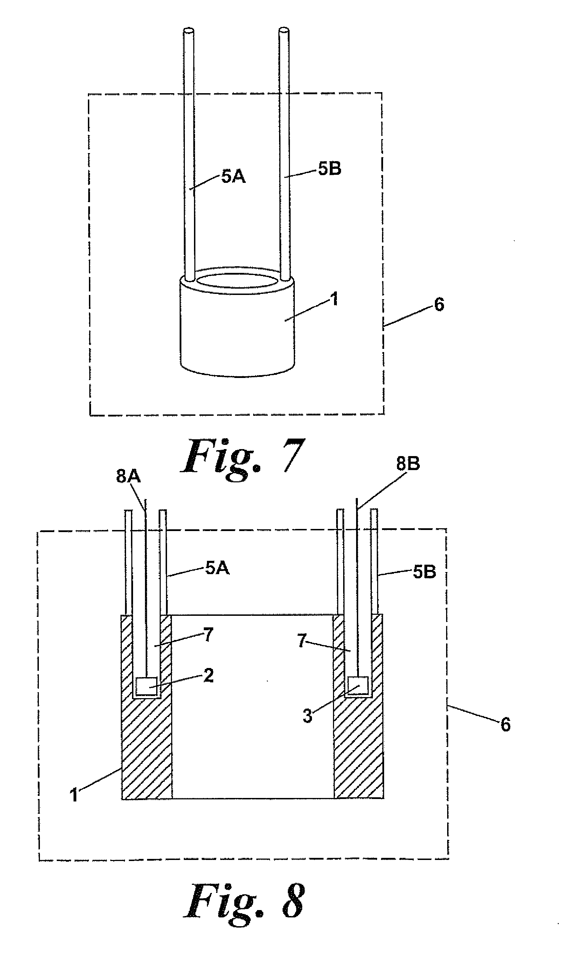

Figure 7 shows a particularly preferred embodiment of the probe 1 having two channels 5a and 5b attached to an end region on the probe 1 and extending, in use, outside the envelope of a bioreactor, illustrated schematically by the box 6. The hollow tubes 5a, 5b provide a channel down which ultrasonic transmitters or detectors may be fed from outside the bioreactor 6 to their intended position on or adjacent to flow stabilisation ring 1. The hollow tubes 5a, 5b may be flexible and may extend within or along the outside of a locating device.

Figure 8 is a cross-sectional view of the apparatus of Figure 7 and illustrates the provision of two pockets 7 located within the wall of the probe 1 into which the ultrasonic transmitter 2 and ultrasonic detector 3 may be positioned. In Figure 8, the transmitter and detector are shown in a slightly spaced-apart relationship with the probe 1, for reasons of clarity. In practice they would be in operable contact with the body of the probe 1 in order to allow transmission of the ultrasonic wave between the transmitter 2 and the detector 3. This may be affected by the use of a tight fit within the pocket, or by use of a coupling medium such as oil or a gel.

By figuring the apparatus in this way, the bioreactor containing the probe as well as the necessary growth medium for the microbial culture may be sterilised, e.g. by heat, before the introduction of the ultrasonic transmitter and detector, which are often heat sensitive. Also illustrated in Figure 8 are the leads 8a and 8b to connect the ultrasonic transmitter to the detector 3 to the respective amplifiers.

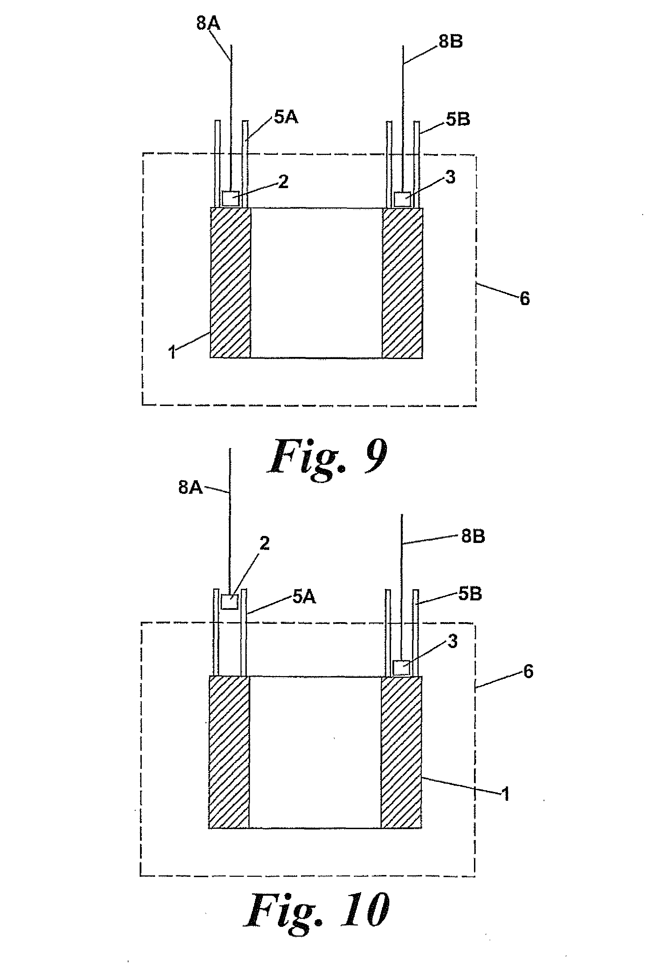

Figure 9 is a cross-sectional view of an alternative configuration of the apparatus of Figure 7, In this embodiment the ultrasonic transmitter 2 and detector 3 are brought into contact with an end face of the probe 1, again through the channels within the tubes 5a and 5b. Figure 10 illustrates how the ultrasonic transmitter 2 and its associated lead 8a maybe withdrawn and replaced outside the envelope of the bioreactor 6. A further advantage of this system, therefore is that the ultrasonic transmitter 2 and/or detector 3 may be replaced during the course of a fermentation e.g. following a failure or may be substituted with an ultrasonic transmitter or detector of a different specification, more appropriate to the particular phase of the fermentation.

With the transmitter 2 and detector 3 located on the sides of the probe 1, the path of the ultrasonic wave between the two has a length equal to the diameter of the probe 1, in this example 30mm, and depth of 30mm (equal to the thickness of the ring, dimension "L", in Figure 4). Generally, the inventor has found that the use of a probe defining a volume of approximately 20ml within the flow path of the probe is particularly appropriate, although smaller or larger volumes may be employed, depending on the size and geometry of the bioreactor vessel to be monitored.

It is envisaged, however, that other dimensions of ring diameter and depth may be required for different fermentation and bio-polymer systems depending on the size of the bio-reactor vessel. This testing procedure may also be applied to very thin liquid films for surface rheology applications, and scale-down studies of bioprocess development (using ultrasonic transducers of suitable size).

In use, the transmitter 2 will be excited, and hence measurements taken by the detector 3 at intervals throughout the fermentation. The timing of intervals between measurements may be varied depending on the current process. For example, more frequent samples may be taken at times of rapid growth than when growth has peaked.

The output signal of the detector 3 is recorded on a data-logging device such as an oscilloscope 14 and logged on to a computer 13. The computational device is programmed to determine the attenuation (amplitude loss) of the ultrasonic wave from the signal generated by the detector 3 and to calculate the viscosity from the attenuation of the ultrasonic wave.

The viscosity and particle size can be calculated using the ECAH equations, which are derived from wave equations which describe the propagation of sound waves of a particular frequency, as follows:

$$(\nabla^2 + k_c^2)\phi_c = 0,$$
$$(\nabla^2 + k_T^2)\phi_T = 0,$$
$$(\nabla^2 + k_s^2)A = 0,$$

$$k_c = \omega/c + i\alpha_L,$$
$$k_T = (1+i)(\omega/2\sigma)^{\frac{1}{2}},$$
$$k_s = (\omega^2\rho/\mu)^{\frac{1}{2}}.$$

• Equation 1

In these equations, k is wavenumber, the reciprocal of wavelength. The subscripts are as follows.

- "c" refers to pressure or compression.
- "s" refers to shear.
- "T" refers to thermal.

Other symbols have the following meanings.

- "$\phi$" and "A" are potentials.
- "$\omega$" is frequency in radians per second.
- "$\rho$" is density.
- "$\sigma$" is shear stress.
- "$\mu$" is a Lamé constant, a constant appearing in elasticity theory (see Sokolnikoff, I, S., 1992, Mathematical Theory of Elasticity, Krieger Publishing Company, ISBN 0898745551.
- "$\alpha_L$" is the damping coefficient for longitudinal, or pressure, waves.

Solving these equations typically involves the method of Frobenius (see Bell, W. W., 1968, Special Functions for Scientists & Engineers, Dover Publications Inc.; Republished), in which solutions are obtained as series expansions in sets of orthogonal functions known as "special functions". Expansions in Legendre Polynomials (Bell, W. W., 1968, Work Cited, Chapter 3) and Bessel and Hankel Functions (Bell, W. W., 1968, Work Cited, Chapter 4) are the most common in this application, although Allegra and Hawley also use the less well known Heine functions ([1] Bell, W. W., 1968, Work Cited, Chapter 3), which are actually better known as spherical Bessel functions.

Having solved these equations, Allegra and Hawley find the following expression for $\alpha_L$:

$$\alpha_L = \frac{-3\varepsilon}{2k_c^2 R^3} \sum_{n=0}^{\infty}(2n+1)\operatorname{Re}(A_n).$$

• Equation 1

In Equation 1,

- "R" is suspended particle radius.
- "$\varepsilon$" is volume fraction of (non-interacting) suspended particles.
- The coefficients "$A_n$" are defined below, and "$\operatorname{Re}(A_n)$" means the real part of the complex numbers $A_n$.

The apparatus is arranged such that the wavelength of the ultrasonic waves is much greater than suspended particle radius. In that case, only the first two terms in the series in Equation 1 are important, and it simplifies to

$$\alpha_L = \frac{-3\varepsilon}{2k_c^2 R^3} \Re(A_0 + 3A_1)$$

● Equation 2

The coefficients $A_0$ and $A_1$ are given below.

$$A_0 = -i\frac{a_c}{3}\left(a_c^2 - \frac{\rho a_c'^2}{\rho'}\right) + \left[\frac{ia_c(c\beta\omega)^2 T_0}{\gamma C_p\left[\omega^2 - \left(\frac{c^2}{\gamma} - \frac{4i\omega\eta_s}{3\rho}\right)k_T^2\right]}\right]\left\{\frac{a_T h_1(a_T)\left(1 - \frac{\rho\beta'T_0\omega^2 C_p}{\rho'\beta T_0\omega^2 C_p'}\right)\left(1 - \frac{\kappa\gamma c'^2\beta'\left[\omega^2 - \left(\frac{c^2}{\gamma} - \frac{4i\omega\eta_s}{3\rho}\right)k_T^2\right]}{\kappa'\gamma'c^2\beta\left[\omega^2 - \left(\frac{c'^2}{\gamma'} - \frac{4i\omega\eta_s'}{3\rho'}\right)k_T'^2\right]}\right)}{h_0(a_T) - \left(\frac{\kappa a_T}{\kappa'a_T'}\frac{h_1(a_T)}{j_1(a_T')}\right)j_0(a_T')}\right\}$$

● Equation 3

In Equation 3, the prime indicates that the property in question refers to the suspended particle. Absence of a prime indicates that the quantity is either universal (such as $i = (-1)^{1/2}$ or R, the particle radius) or that it refers to the host medium in which the particles are suspended.

Other symbols have the following meanings, and may be given primes if necessary.

- "c" is sound velocity.
- $a_c = k_c R = \omega R/c$ = wavenumber times particle radius for pressure waves.
- $a_T = k_T R = (1+ i)(\omega/2\sigma)R$ = wavenumber times particle radius for thermal waves.
- "β" is thermal dilation.
- "ω" is frequency in radians per second.
- "ρ" is density.
- $T_0$ =is mean temperature.
- "γ" is ratio of specific heats.
- "$C_p$" is specific heat at constant pressure.
- "σ" is thermal diffusivity.
- "K" is thermal conductivity.
- "K" is bulk modulus.
- "η" is coefficient of shear viscosity.
- $\lambda = K + (2/3)i\omega\eta$.
- "$\alpha_L$" is the damping coefficient for longitudinal, or pressure, waves.
- $j_0$ and $j_1$ are spherical Bessel functions (also known as Heine functions).
- $h_0$ and $h_1$ are spherical Hankel functions (also known as Heine functions or spherical Bessel functions of the second kind.).

The second term,

$$A_1 = \frac{-i\frac{a_r^2}{3}\left(\frac{\rho}{\rho'}-1\right)\left\{h_2(a_r)\left[a'_rj_1(a'_r)-2\left(1-\frac{\eta_r}{\eta_s}\right)j_2(a'_r)\right]-\left(\frac{\eta_r}{\eta_s'}\right)a_rh_1(a_r)j_2(a'_r)\right\}}{\left[3\left(\frac{\rho}{\rho'}\right)h_2(a_r)-2\left(\frac{\rho}{\rho'}-1\right)h_0(a_r)\right]\left[a'_rj_1(a'_r)-2\left(1-\frac{\eta_r}{\eta_s'}\right)j_2(a'_r)\right]-\left(\frac{\eta_r}{\eta_s'}\right)a_rh_1(a_r)j_2(a'_r)\left(\frac{\rho}{\rho'}+2\right)}$$

• Equation 4

In Equation 4, the prime indicates that the property in question refers to the suspended particle. Absence of a prime indicates that the quantity is either universal (such as $i = (-1)^{1/2}$ or R, the particle radius) or that it refers to the host medium in which the particles are suspended.

Other symbols have the following meanings, and may be given primes if necessary.

- "c" is sound velocity.
- $a_c = k_c R = \omega R/c$ = wavenumber times particle radius for pressure waves.
- $a_s = k_r R = (1+ i)(\omega/2\sigma)R$ = wavenumber times particle radius for thermal waves.
- "β" is thermal dilation.
- "ω" is frequency in radians per second.
- "ρ" is density.
- $T_0$ = is mean temperature.
- "γ" is ratio of specific heats.
- "$C_\rho$" is specific heat at constant pressure.
- "σ" is thermal diffusivity.
- "κ" is thermal conductivity.
- "K" is bulk modulus.
- "η" is coefficient of shear viscosity.
- $\lambda = K+(2/3)i\omega\eta$.
- "$\alpha_L$" is the damping coefficient for longitudinal, or pressure, waves.
- $j_0$ and $j_1$ are spherical Bessel functions (also known as Heine functions).
- $h_0$ and $h_1$ are spherical Hankel functions (also known as spherical Bessel functions of the second kind, or again as Heine Functions) .

The parameters in which the apparatus is trying to measure changes include R, the particle size, ε the volume fraction (as bacteria multiply) and $\eta$,, the viscosity of the host medium. The others are predetermined either by measurement or by estimation.

The computational device is arranged to solve equations 4 and 5 for R and $\eta_s$.

They are not algebraic equations, but transcendental ones because of the functions j and h. Therefore, a numerical method must be used to find the solution. One method is use of an iterative algorithm, two of which are the well-known Newton-Raphson method Scheid, F. (1968, Schaum's Outline of Theory & problems of Numerical Analysis, McGravv Hill, p.310), and the less well known but very reliable method of Kantaris and Howden (Kantaris, N. and Howden, P.F., 1984, The Universal Equation Solver: A Simple New Method for Microcomputers, Sigma Press, ISBN 090514440) .

However, this equation technique assumes no acoustic interaction between the particles suspended in the liquid medium.

In an alternative arrangement, the computational device is arranged to calculate the shear viscosity from the attenuation using finite particle analysis.

In either case, an inventive aspect is to assign a viscoelasticity to the liquid medium. This viscoelasticity is not the true viscoelasticity of the liquid medium but represents the particle to particle interaction that causes the culture to behave, on a macroscopic scale, as a viscoelastic or shear thinning (pseudo-plastic) medium. To the viscosity, $\eta$., of the liquid medium is added a term corresponding to the real part of the viscoelastic modulus (storage & loss viscosity ($\eta'$, $\eta''$)). Hence, η, is modified to become n*, a complex (or dynamic viscosity).

Particular features and benefits of the system described herein are as follows:

The present invention can monitor the physiological status of the microbial system, in terms of bulk viscosity, and elasticity, and hence may be used to control a fermentation process. The elastic moduli (G', E), and the viscosity (loss modulus) have been evaluated as representative indicators of morphology and hence metabolic state of microbial systems as they depict cell-cell and cell-substrate interactions.

Having, identified critical points to control, the system can provide measurements to use for corrective actions to be taken through the development of appropriate algorithms as part of an expert system to control large-scale bioprocesses. The critical points are often expressed in term of physiology indicators, such as bulk viscosity, structure elasticity, sedimentation rate, mean agglomerate size related to growth rate, biomass volume fraction, cell-cell interactions/morphology, substrate-cell surface interaction index, and oxygen uptake. They can be potentially used for bioprocess modelling, and optimisation.

These physiology indicators depict the morphology, and colloidal properties, and hence the metabolic status of the cells as follows:

I) For fungal fermentations, the onset of hyphal tip clumping leads to higher viscosity (usually with shear-thinning behaviour), as well as the formation of pellets with higher radius and sedimentation rate.
II) For cultivation of yeast and non-encapsulated bacterial cells, the kinetics of growth leads to a higher viscosity as a function of hydrodynamic volume fraction.
III) For encapsulated bacterial strains, that result in the building of weak gel structures as bio-films, the loss tangent, and the values of G' are indicative of the cell wall/glyco-calyx interactions in the bio- system. These cells secrete exo-polysaccharides either in the form of "loose slime" or bound to the cell wall.
IV) For mammalian cells growing in vitro in bioreactors, growth generally exhibits Non-Newtonian, shear thinning behaviour, with increasing viscosity during the growth phase.

In the description that follows, the terms "fermenter" and "fermentation" are used with their broad meanings of bioreactor and bioprocess, and are not intended to limit the application to the stricter interpretation of fermentation, i.e. energy-yielding anaerobic metabolic breakdown of a nutrient molecule.

The invention comprises a system for the generation of an ultrasound wave to be continuously propagated through a sample of certain size in-situ e.g. preferably within the envelope of a bioreactor, rather than required a sample to be removed, or to be routed through an external sensing loop) at certain locations in the fermenter (bio-reactor) as we as a system for data collection, and processing. The invention provides a non-destructive testing procedure, hence it "captures" real-time process state without introducing any mechanical disturbance, turbulence or additional eddies in the wave propagation plane.

In various modes of operation, the invention can comprise the application of a continuous or a pulsed ultrasonic wave at intervals during the propagation of the microbial culture by letting the wave propagate in a sample flowing through the ring (cylindrical shaped) loading cell (the probe 1), So the testing procedure ensures a representative sample is tested over time intervals. These locations may be selected as representative of the flow patterns, and of the shear rates applied to the fermentation broth. The probe may also be mounted to a robotic arm capable of changing and adjusting its position as immersed into the broth so measurements can be taken at varying depths in-situ,

During the course of monitoring the state of the bioprocess, the operating mode may be switched from the use of an initial continuous ultrasonic wave (to measure the attenuation of the wave) to the use of a pulsed ultrasonic wave (to measure both the attenuation and speed) as the viscoelastic properties of the medium develop.

Through the propagation of the ultrasonic wave, the system can measure the attenuation ,of the input wave from the output signal. The test can be performed using either the continuous propagation of the wave or the pulse mode. In the latter case, the sound velocity can be calculated thus allowing the calculation of elastic properties.

A particularly preferred operating mode is an ultrasonic frequency of about 5MHz. (say 4.5-5.5MHz) . This avoids diffraction effects of the wave propagation. A fairly large sample path in relation to the vessel diameter is also used, i.e. 3-5cm (30-50mm) to reduce backscattering, The system operates within the region where attenuation coefficient is mainly influenced by viscous damping, hence effects due to scattering are negligible, and hence are kept separate from the bulk viscosity contribution.

The input wave may be transmitted in longitudinal (compressive mode) or perpendicular (shearing mode) depending on the application.

The attenuated output signal is amplified and, if needed, a filter aligned to the detector allows for its de-noising.

Additional software-based refining of the signal may be provided after the amplification step so that the obtained attenuation to be due to the absorption contribution of the microbial cells, and possibly bio-polymer particles may be calculated.

Prior to use, the apparatus should be calibrated. A suitable calibration procedure is as follows: The amplitude of output signal from pulses obtained using deionised water within the fluid stabiliser may be used as reference equivalent to the base line of an instrument. Output signals can be compared for attenuation by solutions containing individual growth medium components at concentrations similar to those in the fermentation medium used for the model fermentation system, e.g. Streptomyces cultures, in order to assess the effects of media components on ultrasound attenuation.

Control samples or particular use are solutions of starch, glucose, minerals, soluble protein digests, and insoluble protein , e.g. cotton seed protein. A growth medium such as Pharmamedia® may be used as typical of a complex industrial media.

Solutions should be tested for ultrasound attenuation before and after heat treatment at e.g. 120°C (a typical temperature for heat sterilisation), and acoustics measurements taken at appropriate operating temperatures for the culture to be characterised, e.g. 25°-35°C to simulate the fermentation conditions.

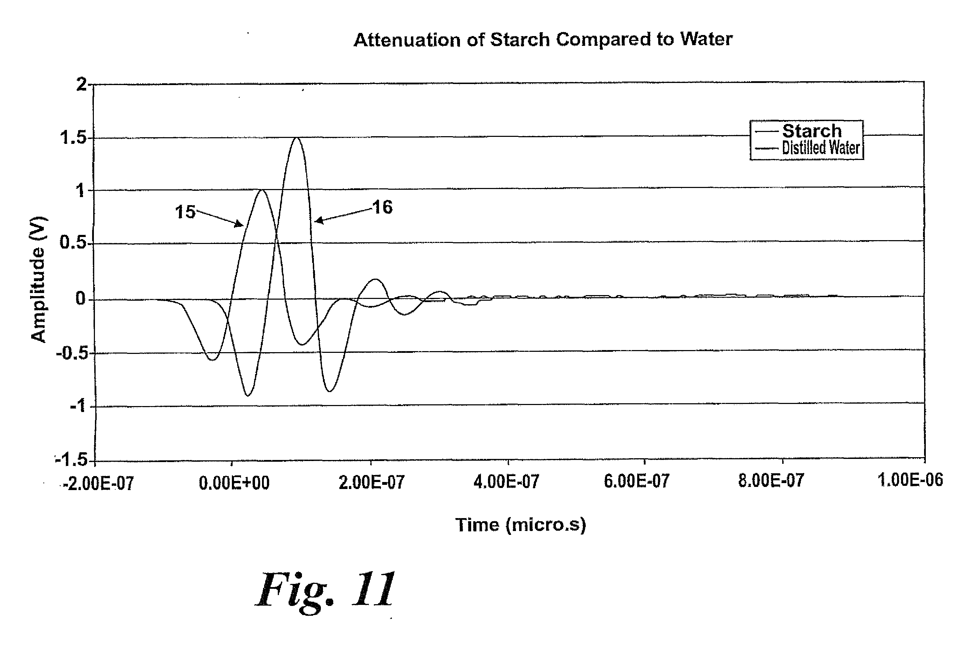

Figure 11 illustrates the attenuation of an ultrasonic wave by a starch solution (15g/litre) by comparison with attenuation by distilled water. It can be seen that the received ultrasonic signal having passed through the starch solution 15 is significantly smaller than that the signal that has passed through the distilled water 16.

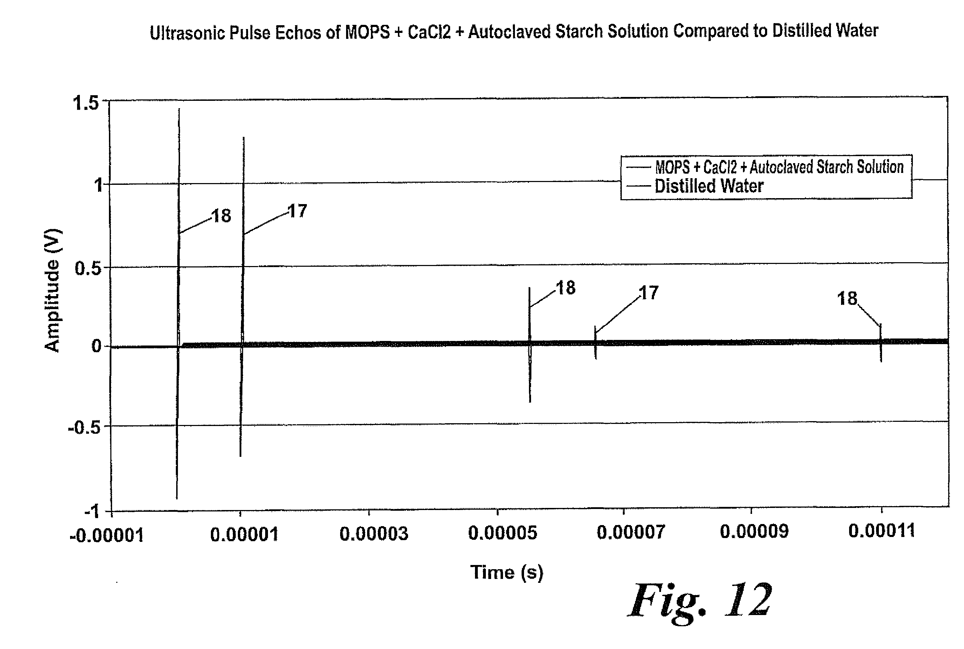

Figure 12 illustrates results of an alternative configuration where an ultrasonic pulse echo technique has been employed, again comparing signals that have passed through a 15g/litre starch solution containing CaC12 and MOPS 17 with that having passed through distilled water 18. Ultrasonic pulse echo techniques, using a single ultrasonic transceiver and a reflecting surface may be employed in the technique as an alternative to the two-transducer system described below.

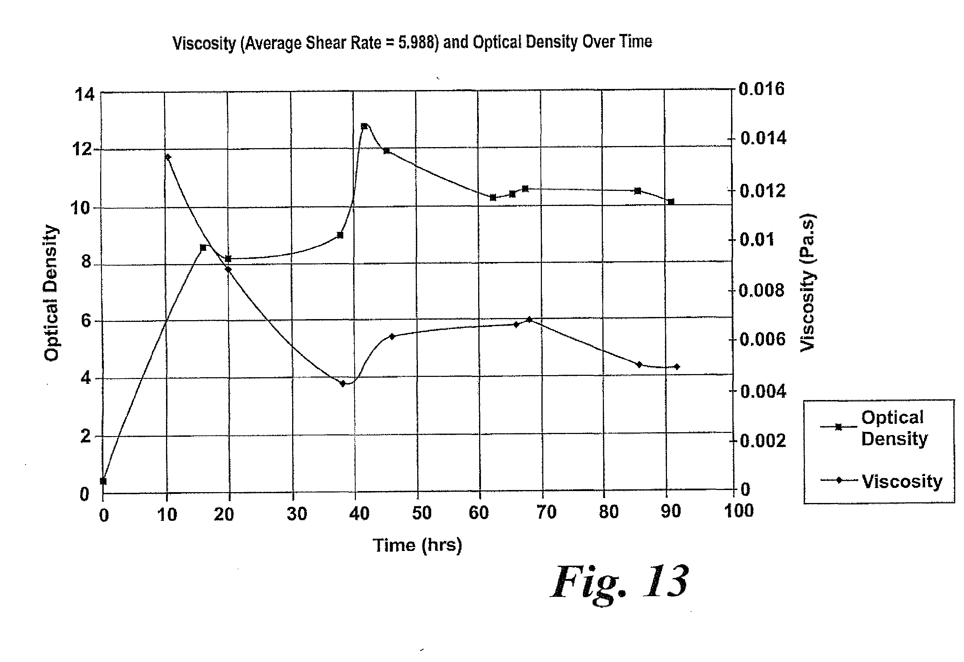

The probe may be calibrated, using viscosity values of standard fermentation broths of Streptomyces species (wild strains of Streptomyces coelicolor and Streptomyces venezuelae) using off-line stepped flow tests at known shear rates. In this way, the attenuation values can be compared, and modelled based on fundamental rheological characterisation of multi-phase fermentation suspensions. Figure 13 shows changes in viscosity during a typical cultivation of Streptomyces venezuelae and changes in the corresponding optical density - an indicator of cell growth.

The attenuated amplitude, and phase angle differences recorded may be converted to the Young modulus (E), and bulk (shear) viscosity (n) by use of the equations presented.

As part of a calibration regime, the attenuation of output signals may be measured using reference samples such as distilled water, aerated water samples, media solutions, complex media containing insoluble polymers, e.g, proteins, and centrifuged biomass harvested from broths (dispersed & pelleted growth) at exponential phase.

In an alternative configuration, the wave can also be propagated in a very thin film of liquid enabling its use for foaming and interfacial studies. In this way, it provides a method for the continuous monitoring of rheological evolution of industrial fermentation broths.

The fact that measurements obtained from specific locations as representative of shear damage or aggregation of particles gives an advantage over previous published inventions which were propagating the wave by pumping and re-circulating sample out of the fermenter, as well as using a certain volume closed cells. Hence, the present invention overcomes the problem of obtaining a representative sample and altering the shear and flow mode of the sample.

In the present invention, the Allegra/Hawley equations may be used to calculate viscosity from amplitude attenuation values that have been obtained experimentally. However, the present invention suggests that the measurement of elastic modulus, and viscosity is a more representative method of evaluating the physiological status of the cell, and hence detecting "critical states" in a typical fermentation process. The bulk rheological changes can be considered as physiology and morphology indicators for they can reveal characteristics such as mass transfer coefficients, elasticity and shear thinning of the biomass, i.e. the strength and extent of cell-cell clumping during exponential phase. The flow regions in the vessels have to be considered separately in the case of heterogeneous fermentation fluids. Most fungal broths are shear thinning so stress calculations based on Doppler shift values from measurements taken near the vessel wall and boundary layer as described in previous invention may not give representative viscosity values for the whole broth.

**Claims**

1. A method of monitoring the state of a microbial culture comprising the steps of: passing an ultrasonic wave through the culture, determining attenuation of the ultrasonic wave by the culture, and calculating an effective viscosity of the culture from the attenuation so determined.

2. A method according to claim 1, wherein changes in the viscosity determined for the culture are used to determine a state of the microbial culture.

3. A method according to claim 1 or claim 2, wherein the culture comprises a suspension of particles in a non-viscoelastic liquid medium and the effective viscosity of the culture is determined using an algorithm, wherein a term is added equivalent to viscoelacticity for the liquid medium.

4. A method according to claim 3 wherein the culture comprises a suspension of particles in a non-viscoelastic liquid medium, wherein the effective viscosity of the culture is determined using an iterative algorithm based on the ECAH equations, finite element analysis or boundary element analysis, wherein a term is added equivalent to a viscoelacticity for the liquid medium.

5. A method according to claim 3 or claim 4, wherein added to the viscosity of the liquid medium is a term corresponding to the real part of a viscoelastic modulus.

6. A method according to any one of the preceding claims, comprising passing an ultrasonic wave through the culture, wherein the wavelength of the ultrasonic wave is greater than the size of the particles of the culture.

7. A data carrier having instructions thereon that when executed by a processor causes the processor to receive a measurement of intensity for an ultrasonic wave that has been passed through a microbial culture, determine an attenuation of the ultrasonic wave by the culture; and calculate an effective viscosity of the culture from the attenuation so determined.

8. Apparatus for monitoring the state of a microbial culture in a bioreactor comprising an emitter for generating an ultrasonic wave such that the ultrasonic wave passes through culture in the bioreactor, a detector for measuring the intensity of ultrasonic waves that have passed through the culture, and a computational device arranged to receive signals indicative of the measurement by the detector, determine from the signal the attenuation of the ultrasonic wave; and calculate an effective viscosity of the culture from the attenuation so determined.

9. Apparatus according to claim 10, wherein the culture comprises a suspension of particles in a non-viscoelastic liquid medium and the computational device determines the effective viscosity of the culture using an algorithm, wherein a term is added equivalent to viscoelacticity of the liquid medium.

10. Apparatus according to claim 8 or claim 9, wherein the computational device is programmed to calculate the viscosity of the culture using an iterative algorithm based on the ECAH equations, finite element analysis or boundary element analysis.

11. Apparatus according to claim 8 to 10, wherein the transmitter is arranged to generate ultrasonic waves having a wavelength less than or of the order of the size of the particles of the culture.

*Fig. 1*

*Fig. 2*

*Fig. 3a*

**Fig. 3b**

39

35

42

34

40

22

**Fig. 4a**

36

37

38

*Fig. 4b*

## Fig. 5

## Fig. 6

**Fig. 7**

**Fig. 8**

**Fig. 9**

**Fig. 10**

Attenuation of Starch Compared to Water

*Fig. 11*

EP 2 506 004 A2

Ultrasonic Pulse Echos of MOPS + CaCl2 + Autoclaved Starch Solution Compared to Distilled Water

*Fig. 12*

EP 2 506 004 A2

Viscosity (Average Shear Rate = 5.988) and Optical Density Over Time

*Fig. 13*

EP 2 506 004 A2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02071050 A
- US 6378357 B1
- US 20050132784 A1
- US 20050126268 A1
- US 20050150275 A1

**Non-patent literature cited in the description**

- **EPSTEIN, P. S. ; CARHART, R. R.** The Absorption of Sound in Suspensions and Emulsions: I:Water Fog in Air. *J. Acoust. Soc. Amer,* 1953, vol. 25, 553
- **MORSE, P. M. ; FESHBACH, H.** Methods of Theoretical Physics. 1953, vol. 2, 1432-1512
- **ALLEGRA, J. R. ; HAWLEY, S. A.** Attenuation of Sound in Suspensions and Emulsions. *J. Acoust. Soc. Amer.,* 1972, vol. 51, 1545-1564
- **VERDIER, C. ; PIAU, M.** Acoustic Wave Propagation in Two-Phase Viscoelastic Fluids: the Case of Polymer Emulsions. *J. Acoust. Soc. Amer.,* 1997, vol. 101, 1868-1876
- **HIPP, A. K. ; ADJADJ, L. P. ; STORTI, G. ; MORBIDELLI, M.** *J: Acoust. Soc. Amer,* 2002, vol. 111 (4), 1549-1551
- **FERRY, J. D.** Viscoelastic Properties of Polymers. John Wiley & Sons Inc, 1980
- **DUKHIN, A.S. ; GOETZ, P. J.** Ultrasound for Characterising Colloids. Elsevier, 2002
- **SOKOLNIKOFF, I, S.** Mathematical Theory of Elasticity. Krieger Publishing Company, 1992
- **BELL, W. W.** Special Functions for Scientists & Engineers. Dover Publications Inc,
- **BELL, W. W.** Expansions in Legendre Polynomials. 1968
- **BELL, W. W.** Bessel and Hankel Functions. 1968
- Schaum's Outline of Theory & problems of Numerical Analysis. McGravv Hill, 1968, 310
- **KANTARIS, N. ; HOWDEN, P.F.** The Universal Equation Solver: A Simple New Method for Microcomputers. Sigma Press, 1984